# FASCICULE DE BREVET EUROPEEN

(11) **EP 1 877 035 B1**
(45) Date de publication et mention de la délivrance du brevet: **14.12.2016**
(21) Numéro de dépôt: 06709077.9
(22) Date de dépôt: 12.01.2006
(51) Int. Cl.: A61K 9/00, A61K 9/20

(54) **COMPOSITION PHARMACEUTIQUE ET FORME GALÉNIQUE CORRESPONDANTE À DÉLITEMENT RAPIDE EN BOUCHE, ET PROCÉDÉ DE FABRICATION DE CETTE COMPOSITION**
IN DER MUNDHÖHLE RASCH ZERFALLENDE ARZNEIZUSAMMENSETZUNG UND DOSIERUNGSFORM, UND VERFAHREN ZU IHRER HERSTELLUNG
PHARMACEUTICAL COMPOSITION AND DOSAGE FORM FAST-DISINTEGRATING IN THE MOUTH , AND PROCESS TO PREPARE IT

(30) Priorité: 12.01.2005 EP 05290077
(43) Date de publication de la demande: 16.01.2008
(73) Titulaire: Physica Pharma, 33650 Martillac (FR)
(72) Inventeur: BROUSSAUD, Olivier, F-33000 BORDEAUX (FR); POUGNAS, Jean-Luc, F-33500 LIBOURNES (FR); CALVET, Nicolas, F-33200 BORDEAUX (FR)
(74) Mandataire: Richaud, Fabien
(86) Numéro de dépôt international: PCT/FR2006/000070
(87) Numéro de publication internationale: WO 2006/075097

(56) Documents cités:
- EP-A- 0 208 213
- WO-A-99/03449
- WO-A-02/089775
- WO-A-2004/019918
- US-A- 5 023 108
- US-A- 5 288 501

## Description

La présente invention concerne une composition pharmaceutique pulvérulente utilisable pour constituer après compression une forme galénique à délitement rapide en bouche, une telle forme galénique et un procédé de fabrication de cette composition pharmaceutique.

Le développement de compositions pharmaceutiques destinées à constituer des formes galéniques solides qui, lorsqu'elles sont administrées dans la bouche de patients ayant des difficultés pour déglutir, tels que des enfants, des personnes âgées, des personnes mentalement déficientes ou non coopératives médicalement, sont aptes à se déliter (i.e. à se désagréger ou à se désintégrer) rapidement sans nécessiter un apport d'eau, suscite de nos jours un intérêt croissant.

Par « forme galénique solide à délitement rapide en bouche », on entend de manière connue, comme défini par la Pharmacopée Européenne, un comprimé non pelliculé (i.e. non enrobé) qui, lorsqu'il est introduit en bouche, se désintègre en moins de trois minutes avant d'être avalé. Le temps nécessaire à cette désintégration est communément appelé vitesse d'hydratation du comprimé.

Différentes formes galéniques solides à délitement rapide en bouche sont largement utilisées à ce jour. Il s'agit essentiellement de lyophilisats oraux, de comprimés effervescents oraux, ou encore de comprimés à forte teneur en agents de délitement pour l'obtention d'un délitement immédiat après introduction en bouche.

Les lyophilisats oraux ou « freeze drying tablets » représentent les compositions à délitement rapide les plus utilisées, notamment du fait qu'ils permettent d'assurer une désintégration éclair 10 secondes environ après leur administration en bouche, en raison de leur hygroscopicité et de leur porosité élevées. Néanmoins, ces lyophilisats présentent les inconvénients d'être très fragiles lors de leur manipulation en raison de leur friabilité élevée, d'être extrêmement sensibles à l'humidité atmosphérique et d'impliquer une technologie et un procédé de fabrication complexes et, par conséquent, des coûts de fabrication élevés. De ce fait, cette technologie est généralement retenue pour des principes actifs coûteux et faiblement dosés dans la composition pharmaceutique.

Les comprimés effervescents oraux sont constitués d'une composition pharmaceutique comprenant un couple effervescent, lequel entre en action dès son contact avec la salive, assurant ainsi le délitement complet du comprimé. Cependant, ces comprimés effervescents doivent nécessairement présenter une taille réduite afin de garantir un délitement en bouche inférieur à 3 minutes, de limiter la quantité de gaz émis et de limiter le mauvais goût en bouche qui les caractérise. Un autre inconvénient de ces comprimés effervescents est qu'ils sont très sensibles à l'humidité, ce qui implique l'utilisation d'un procédé de fabrication très particulier, typiquement en atmosphère contrôlée.

Les comprimés à forte teneur en agents de délitement sont par exemple décrits dans le document de Brevet US-A-5 464 632. Ce document divulgue une composition pharmaceutique qui comprend notamment, à titre d'agents de délitement associés à des agents de gonflement, une fraction massique élevée de carboxyméthylcellulose et d'une polyvinylpirrolidone réticulée et insoluble. Cette composition est obtenue par mélangeage d'un principe actif, utilisé sous forme de microcristaux ou de microgranules, avec un mélange d'excipients préalablement soumis à une granulation.

Cette fraction massique élevée d'agents de délitement dans les compositions selon ce document présente l'inconvénient de générer un goût de type crayeux et une sensation de sécheresse indésirable, dès l'introduction en bouche de ces comprimés les incorporant.

La polyvinylpirrolidone réticulée mentionnée précédemment est également utilisée en tant qu'agent désintégrant (agent de délitement) dans les suspensions aqueuses (ou « aqueuses/organiques ») objet du brevet US 5,288,501, comprenant également, entre autres, un alcool polyvinylique spécifique ; lesdites suspensions étant utilisées comme revêtement pour des particules contenant, en leur sein, un principe actif.

La plupart des excipients utilisés dans les compositions de comprimés à délitement rapide sont en général très solubles en milieu aqueux ou bien présentent un fort pouvoir de gonflement, typiquement dans le cas de l'utilisation d'agents de délitement.

Afin d'améliorer les sensations en bouche des comprimés, on a développé en particulier des compositions contenant majoritairement des excipients très solubles dans l'eau, tels que des polyols, du mannitol, du xylitol ou du sorbitol, la grande solubilité de ces excipients favorisant un délitement rapide des comprimés dès leur contact avec la salive. Néanmoins, l'utilisation de ces excipients, tels que des polyols, présente l'inconvénient d'augmenter les risques de collage lors de l'étape de compression et de ne pas réduire la sensibilité des comprimés à l'humidité ambiante.

Le document de Brevet WO-A-03/086361 préconise la mise en oeuvre d'une granulation par voie humide pour l'obtention d'une composition pharmaceutique destinée à former un comprimé à délitement rapide en bouche. Dans les exemples de ce document, cette granulation est réalisée via une pulvérisation d'une solution aqueuse de lauryl sulfate de sodium sur un mélange pulvérulent comprenant un principe actif dispersé dans des excipients.

Un inconvénient majeur de la granulation qui est mise en oeuvre dans ce document réside dans la densification des particules de poudre qu'elle implique, laquelle pénalise la pénétration de la salive à coeur du granulé obtenu même avec l'ajout de surfactant, ce qui va à l'encontre d'un délitement rapide des comprimés.

D'une manière connue, le développement de comprimés à délitement rapide en bouche doit prendre en compte un ensemble de paramètres physiques qui sont généralement contradictoires, incluant, pour les comprimés obtenus :
- une friabilité réduite et de préférence inférieure à 1 %, sinon la manipulation des comprimés peut devenir problématique tant lors de leur fabrication (risque d'endommagement des comprimés lors de l'étape de mise en emballage ou « blister », notamment) et lors de l'intervention de l'utilisateur final (risque d'écrasement des comprimés lors de l'extraction du « blister » et d'effritement des comprimés dans la main de l'utilisateur),
- une dureté ou résistance à la rupture qui est au contraire relativement élevée, étant typiquement d'au moins 15 Newtons, et
- une vitesse d'hydratation en bouche inférieure à trois minutes, cette vitesse étant généralement pénalisée en cas de dureté trop élevée des comprimés.

Un but de la présente invention est de remédier aux inconvénients précités, et ce but est atteint en ce que la Demanderesse vient de découvrir d'une manière surprenante que la pulvérisation d'une solution colloïdale aqueuse comprenant un système auto-émulsionnable incluant en combinaison au moins un composé filmogène hydrophile, au moins un composé amphiphile lubrifiant et au moins un composé amphiphile humectant, sur des particules solides à base d'un mélange d'excipients comprenant au moins un diluant, de telle manière que le rapport massique (solution colloïdale pulvérisable/ particules destinées à recevoir cette solution colloïdale) soit inférieur ou égal à 10 %, permet d'obtenir après séchage une composition pulvérulente qui est constituée desdites particules solides revêtues d'un film pré-hydratant à base de ladite solution colloïdale et qui est utilisable pour constituer après compression un comprimé présentant une vitesse d'hydratation (i.e. de délitement en bouche) nettement inférieure à 60 secondes et même inférieure à 30 secondes.

A titre de solution colloïdale aqueuse utilisable pour constituer ledit film, on utilise une solution ou suspension de type huile dans eau ou bien eau dans huile (par exemple de type microémulsion) comprenant, d'une part, une phase aqueuse et, d'autre part, ledit système auto-émulsionnable.

Par système auto-émulsionnable, on entend de manière connue un système permettant à l'émulsion de se former et de se reformer spontanément sans agitation mécanique ni apport d'énergie thermique. De cette manière, ladite solution colloïdale est apte à se reformer spontanément lors de la mise en contact avec la salive dudit film recouvrant lesdites particules solides.

Selon l'invention, on notera que cette pulvérisation ne constitue en aucune manière une granulation (telle que les granulations par voie humide connues dans l'état de l'art), du fait que les particules revêtues du film pré-hydratant selon l'invention sont libres (i.e. n'adhèrent pas entre elles) et présentent une granulométrie et une densité apparente pratiquement inchangées, en raison du rapport massique précité relativement réduit qui se traduit par l'obtention en surface dudit film d'épaisseur réduite.

En effet, les granulés obtenus par des techniques de granulation sont caractérisés de manière connue par un agglomérat de particules collées les unes aux autres qui est recouvert d'une couche d'enrobage d'épaisseur relativement élevée, à la différence du film mince obtenu selon l'invention.

En d'autre termes, une composition pharmaceutique pulvérulente selon l'invention, qui est utilisable pour constituer après compression une forme galénique à délitement rapide en bouche et qui comprend lesdites particules solides, est telle que ces dernières sont revêtues dudit film pré-hydratant, de telle sorte que lesdites particules ainsi revêtues soient libres les unes par rapport aux autres dans ladite composition (i.e. ces particules ne sont pas collées les unes aux autres, du fait que la composition est dépourvue de tout liant).

Outre ce délitement accéléré en bouche, on notera que les comprimés selon l'invention présentent avantageusement :
- une dureté ou résistance à la rupture, mesurée selon la Pharmacopée Européenne, qui est supérieure à 15 Newtons et de préférence supérieure à 20 Newtons, et
- une friabilité, également mesurée selon la Pharmacopée Européenne, qui est inférieure à 1 %, ce qui permet de remédier à l'inconvénient précité relatif à la manipulation des comprimés à la fois lors de leur fabrication et lors de l'intervention de l'utilisateur final.

Cette composition pharmaceutique selon l'invention peut comprendre en outre au moins un principe actif à l'état dispersé dans ledit mélange d'excipients, cette dispersion du principe actif pouvant être réalisée après la pulvérisation (i.e. par mélangeage final dudit principe actif et desdites particules à base dudit mélange d'excipients ayant été revêtues dudit film) ou bien avant celle-ci (i.e. par mélangeage préalable dudit principe actif et desdites particules à base dudit mélange d'excipients non encore revêtues).

A titre de diluant(s) utilisable(s) dans ledit mélange d'excipients, on utilise des diluants solubles dans l'eau, le(s)dit(s) diluant(s) étant présent(s) dans ladite composition selon une fraction massique totale allant de 30 % à 90 % et de préférence allant de 50 à 85 %.

Ledit ou l'un au moins desdits diluants solubles dans l'eau est choisi parmi une dextrine, une maltodextrine, le lactose, le saccharose, le tréhalose, le mannitol, le sorbitol, l'érythritol, le maltitol, le lactitol et le fructose.

Selon une autre caractéristique de l'invention, la fraction massique de ladite solution colloïdale à l'état sec dans ladite composition appartient à un domaine allant de 0,01 % à 10 %, de préférence allant de 0,1 % à 5 % et, à titre encore plus préférentiel, allant de 0,5 % à 3 %.

A titre de composé(s) filmogène(s) hydrophile(s), on utilise :
- au moins un carbohydrate choisi dans le groupe constitué par les maltodextrines, les dextrines, le sorbitol, le mannitol et le xylitol, ou
- un polyéthylène glycol de masse moléculaire moyenne en poids Mw allant de 1000 g/mol à 6000 g/mol.

On notera que ledit composé filmogène hydrophile permet d'augmenter la vitesse d'hydratation de la composition pharmaceutique, tout en participant à la cohésion de celle-ci.

A titre de composé(s) amphiphile lubrifiant, on utilise un composé choisi dans le groupe constitué par le lauryl éther sulfate de sodium, le lauryl sulfate de sodium, et le stéarate de magnésium.

A titre de composé(s) amphiphile humectant, on utilise un composé choisi parmi un lauroyl macrogolglycéride, tel qu'un lauroyl macrogol-32 glycéride (Gelucire^{®}44/14), et un stéaroyl macrogolglycéride, tel qu'un stéaroyl macrogol-32 glycéride (Gelucire^{®}50/13).

Ledit mélange d'excipients utilisé dans la composition selon l'invention peut également comprendre, en combinaison avec le(s)dit(s) principe(s) actif(s) et le(s)dit(s) diluant(s), un ou des agent(s) de cohésion (souvent appelés « glidant agents » par l'homme de l'art) tels que, à titre non limitatif, la cellulose microcristalline ou un de ses dérivés, une dextrine telle que la maltodextrine, un lactose, un dérivé de phosphate de calcium ou un dérivé d'amidon, le(s)dit(s) agent(s) de cohésion étant présent(s) dans ladite composition selon une fraction massique allant de 0,1 % à 40 %.

Ces agents de cohésion sont utilisés pour faciliter la fabrication des comprimés et maintenir la friabilité de ces derniers inférieure à 1 %. La fraction massique dans ladite composition selon l'invention du ou des agent(s) de cohésion peut être comprise entre 0,1 % et 40 % et, de préférence, cette fraction massique est inférieure à 20 %.

La composition pharmaceutique selon l'invention peut contenir des proportions variables de principe(s) actif(s), typiquement de 0,1 % à 50 %.

A titre exemplatif, on peut citer :
- des anti-ulcéreux, tels que Famotidine, Oméprazol, Lanzoprazol,
- des anti-émétiques, tels que Ondansetron, Granisetron, Dolasetron, Domperidone, Metoclopramide,
- des antihypertenseurs, tels que Enalapril, Losartan, Candesartan, Valsartan, Lisinopril, Ramipril, Doxazosin, Terazosin,
- des antihistaminiques, tels que Loratadine, Cetirizine,
- des anti-psychotiques, tels que Risperidone, Olanzapine, Quetiapine,
- des anti-dépresseurs, tels que Paroxetine, Fluoxetine, Mirtazapine,
- des analgésiques et anti-inflammatoires, tels que Piroxicam,
- des anti-hypercholestérolémiants, tels que Simvastatin, Lovastatin, Pravastatin,
- des anti-migraineux, tels que Zolmitriptan, Naratriptan, Rizatriptan, Sumatriptan,
- des anti-épileptiques, tels que Lamotrigine,
- des anti-Parkinsoniens, tels que Selegiline, Apomorphine,
- des anxiolytiques, tels que Diazepam, Lorazepam, Zolpidem,
- des anti-asthmatiques, tels que Zafirlukast, Montelukast,
- des agents du dysfonctionnement de l'érection, tels que Sildenafil,
- des anti-pyrétiques, tels que Paracétamol, l'acide acétylsalicylique, Ibuprofène, etc.

On notera que tous ces principes actifs peuvent être utilisés indifféremment sous leur forme base ou sous une forme de sels, d'hydrates, de solvates et d'isomères.

La composition pharmaceutique selon l'invention peut contenir en outre :
- des agents édulcorants, tels que du saccharinate de sodium ou de l'aspartame,
- des agents aromatisants, et/ou
- des agents lubrifiants, tels que le stéarate de magnésium, l'acide stéarique, le stéaryl fumarate de sodium, le talc, un PEG, ou des dérivés pulvérulents lipidiques comme par exemple le tribéhénate de glycérol.

Une forme galénique selon l'invention, qui présente un délitement rapide en bouche, comprend un comprimé non enrobé (i.e. non pelliculé) qui est constitué de ladite composition pharmaceutique telle que définie ci-dessus.

D'une manière générale, ces formes galéniques selon l'invention sont utilisables pour un traitement thérapeutique et/ou prophylactique d'un organisme humain ou animal.

L'invention concerne également un procédé de fabrication tel que revendiqué dans la revendication 11.

De préférence, on choisit d'incorporer le(s)dit(s) principe(s) actif(s) à la composition pharmaceutique suite à la pulvérisation, comme indiqué à l'étape d), notamment dans le cas ou ledit ou l'un au moins desdits principes actifs est sensible à l'eau.

Selon l'invention, ledit système auto-émulsionnable comprend en combinaison, comme défini ci-dessus, le(s)dit(s) composé(s) filmogène(s) hydrophile(s), le(s)dit(s) composé(s) amphiphile(s) lubrifiant(s), et le(s)dit(s) composé(s) amphiphile(s) humectant(s).

Avantageusement, la solution colloïdale préparée à l'étape a) comprend une fraction massique dudit système auto-émulsionnable qui est inférieure ou égale à 10 %, et une fraction massique d'eau qui est égale ou supérieure à 90 %.

De préférence, la solution colloïdale obtenue à l'étape a) est chauffée à une température allant de 30° C à 70° C, pour la mise en oeuvre de l'étape b) à une telle température allant de 40° C à 70° C.

En effet, la Demanderesse a découvert que cette plage de températures permet avantageusement d'optimiser la pré-hydratation desdites particules solides par un mouillage et une tenue améliorés du film de solution colloïdale pulvérisé, en comparaison d'une pulvérisation réalisée à la température ambiante (par exemple 25°C).

Egalement à titre préférentiel, la viscosité de ladite solution colloïdale à pulvériser à l'étape b) appartient à un domaine allant de 2 mPa.s à 40 mPa.s à 50° C, et les gouttelettes pulvérisées présentent une taille moyenne allant sensiblement de 10 µm à 100 µm et, de préférence, allant de 50 µm à 70 µm. On notera que cette pulvérisation est en fait assimilable à une nébulisation dans le cas spécifique de gouttelettes dont la taille moyenne varie sensiblement de 10 à 50 µm.

On notera également que cette taille réduite des gouttelettes est particulièrement bien adaptée pour l'obtention dudit film mince pré-hydratant selon l'invention qui recouvre en surface lesdites particules solides sans les faire adhérer entre elles.

Selon une autre caractéristique de ce procédé selon l'invention, le rapport de la densité apparente de la composition obtenue à l'étape c) ou d) sur la densité apparente des particules solides avant pulvérisation est compris entre 1 et 1,2.

Selon une autre caractéristique de l'invention, la composition pharmaceutique ainsi obtenue présente, avant compression, une densité apparente qui, mesurée selon la Pharmacopée Européenne au moyen d'un voluménomètre de tassement (via 3 mesures de volumes apparents V₀, V₁₀ et V₅₀₀ - V₁₀ représentatives de l'aptitude au tassement), appartient à un domaine allant de 0,4 à 0,9 g /mL.

De plus, lesdites particules revêtues du film selon l'invention présentent, après pulvérisation et séchage, une augmentation de taille qui est inférieure ou égale à 20 %.

Les caractéristiques précitées de la présente invention, ainsi que d'autres, seront mieux comprises à la lecture de la description suivante de plusieurs exemples de réalisation de l'invention, donnés à titre illustratif.

### EXEMPLES :

### 1) Comprimé 1 « témoin » et comprimé 2 selon l'invention de type placebo :

### a) Préparation des comprimés :

On a préparé des comprimés « témoin » et selon l'invention à délitement rapide qui sont tous de type placebo, i.e. dépourvus de tout principe actif.

Ces comprimés placebo « témoin » et selon l'invention sont respectivement constitués de compositions 1 et 2 pulvérulentes présentant les formulations suivantes à l'état sec (en fractions massiques dans le tableau 1 a ci-après) :

**Tableau 1a :**

| COMPOSITIONS | 1 | 2 |
|---|---|---|
| Particules solides d'excipients : | | |
| PHARMABURST® (diluant à base de mannitol) | 76 % | 75,81 % |
| Arôme fraise | 1 % | 1 % |
| Aspartame | 1 % | 1 % |
| stéaryl fumarate de sodium | 2 % | 2 % |
| Cellulose microcristalline (agent de cohésion) | 20 % | 20 % |
| Film d'une solution colloïdale après séchage | | |
| à base d'eau et du système auto-émulsionnable suivant : | | |
| Maltodextrine (composé hydrophile filmogène) | 0 % | 0,08 % |
| lauryl sulfate de sodium (composé amphiphile lubrifiant) | 0 % | 0,03 % |
| « GELUCIRE 44/14 » (composé amphiphile humectant) | 0 % | 0,08 % |

On notera que la composition 1 « témoin » n'a pas été pré-hydratée, contrairement à la composition 2 selon l'invention qui a été pré-hydratée par pulvérisation, sur les particules solides d'excipients, de la solution colloïdale aqueuse précitée, qui est de type huile dans eau et qui a été séchée en étuve après pulvérisation.

Le tableau 1 b ci-après détaille la formulation utilisée pour l'obtention de cette solution colloïdale aqueuse (fractions massiques) :

**Tableau 1b:**

| | |
|---|---|
| Maltodextrine | 1,6 % |
| Lauryl sulfate de sodium | 0,6 % |
| « GELUCIRE 44/14 » | 1,6 % |
| Eau | 96,2 % |

Cette solution colloïdale filmogène a été chauffée à 50° C puis finement pulvérisée à cette même température selon un volume pulvérisé égal à 5 mL, étant précisé que sa viscosité à cette température était de 3 mPa.s.

De plus, les gouttelettes pulvérisées présentaient une taille moyenne allant sensiblement de 50 µm à 70 µm.

Cette composition 2 obtenue est telle que les particules d'excipients sont revêtues en surface d'un film d'épaisseur réduite, de telle sorte que les particules ainsi revêtues ne forment pas un granulé (i.e. ces particules n'adhèrent pas entre elles en dépit de la pulvérisation et présentent après cette dernière une taille sensiblement identique à leur taille initiale).

Le tableau 2 ci-après mentionne le volume apparent et la densité apparente des compositions 1 et 2 avant compression de celles-ci.

**Tableau 2 :**

| COMPOSITIONS | 1 | 2 |
|---|---|---|
| Volume apparent (cm³) | 190 | 186 |
| Densité apparente (g/cm³) | 0,526 | 0,537 |

On notera que l'absence de granulation, qui caractérise la pulvérisation mise en oeuvre pour l'obtention de la composition 2 selon l'invention, se traduit par une densité apparente de cette composition 2 avant compression qui est quasiment inchangée suite à la pulvérisation et au séchage, i.e. sensiblement égale à 0,526 g/cm³.

Chacun de ces comprimés « témoin » et selon l'invention, de type rond et plat, présente un diamètre de 12 mm, une épaisseur moyenne de 2,8 mm, une masse moyenne finale de 320 mg, une dureté moyenne de 21 Newtons et une friabilité de l'ordre de 0,45 %.

### b) Essais in vitro d'hydratation de ces comprimés :

On a procédé à des essais comparatifs de vitesse d'hydratation portant sur six comprimés 1 « témoin » et sur six comprimés 2 selon l'invention. Les conditions suivies pour ces essais étaient les suivantes :
- volume V de chaque comprimé : 317mm³, avec V= aire de la surface de la partie supérieure plane du comprimé x hauteur du comprimé), et
- volume d'eau déposé sur chaque comprimé : 320 mm³.

On a déposé 320 mm³ d'eau à la surface de chaque comprimé, puis on a mesuré le temps au bout duquel toute l'eau a disparu de la surface des comprimés. On a alors calculé la vitesse d'hydratation, cette vitesse correspondant en fait au temps nécessaire pour qu'un mm³ d'eau soit adsorbé par le comprimé correspondant.

Le tableau 3 ci-après présente les résultats moyens obtenus :

**Tableau 3 :**

| COMPOSITIONS | 1 | 2 |
|---|---|---|
| Temps d'absorption (en secondes) | 33 s | 20 s |
| Vitesse d'hydratation (pour 1 mm³) | 0,102 s/ mm³ | 0,0625 s/ mm³ |

Ces résultats montrent que les comprimés 2 selon l'invention, qui sont obtenus par pulvérisation de ladite solution colloïdale pré-hydratante sur lesdites particules d'excipients sans mettre en oeuvre une granulation, adsorbent l'eau environ 1,6 fois plus vite pour 1 mm³ que les comprimés 1 « témoin » qui sont dépourvus d'une telle solution colloïdale pré-hydratante, ce qui témoigne d'une vitesse d'hydratation nettement améliorée pour ces comprimés 2 selon l'invention.

### 2) Comprimés 3 selon l'invention :

### a) Préparation des comprimés 3 :

On a préparé des compositions pharmaceutiques 3 pulvérulentes selon l'invention d'une manière analogue à celle décrite pour les compositions 2 placebo selon l'invention du § 1) ci-dessus, excepté qu'après la pulvérisation de la solution colloïdale aqueuse sur un pré-mélange de particules solides d'excipients, on a procédé à une dispersion d'un principe actif dans les particules d'excipients revêtues du film de pulvérisation. Chaque composition 3 présente la formulation suivante à l'état sec (en fractions massiques dans le tableau 4a ci-après) :

**Tableau 4a :**

| COMPOSITION | 3 |
|---|---|
| Pré-mélange de particules d'excipients revêtu par pulvérisation : | |
| PHARMABURST® (diluant à base de mannitol) | 78 % |
| Arôme menthe | 1 % |
| Aspartame | 1 % |
| « PRUV » (lubrifiant anti-collant pour la mise en oeuvre) | 4 % |
| cellulose microcristalline « AVICEL Ph 200 » (agent de cohésion) | 10 % |
| Film d'une solution colloïdale selon l'invention après séchage | |
| à base d'eau et du système auto-émulsionnable suivant : | |
| « PEG 4000 » (composé hydrophile filmogène) | 2 % |
| lauryl éther sulfate de sodium (composé amphiphile lubrifiant) | 0,5 % |
| « GELUCIRE 44/14 » (composé amphiphile humectant) | 0,5 % |
| Glycérine | 2 % |
| Rispéridone (principe actif) | 1 % |

Le tableau 4b ci-après détaille la formulation utilisée pour l'obtention de cette solution colloïdale aqueuse (fractions massiques) :

**Tableau 4b :**

| | |
|---|---|
| « PEG 4000 » | 20 % |
| Lauryl éther sulfate de sodium | 5 % |
| « GELUCIRE 44/14 » | 5 % |
| Glycérine | 20 % |
| Eau | 50 % |

Cette solution colloïdale filmogène a été chauffée à 50° C puis pulvérisée en fines gouttelettes sur les particules d'excipients en mouvement à cette même température, selon un volume pulvérisé égal à 10 mL.

On a produit ces fines gouttelettes via une buse de pulvérisation classique de type à cône creux. Des essais ont montré que la durée nécessaire et suffisante pour pulvériser ces 10 mL de solution colloïdale sur les particules d'excipients était de 3 minutes.

Le protocole opératoire suivi était le suivant :
- on a agité le pré-mélange de particules d'excipients à revêtir (ce pré-mélange étant dépourvu de principe actif), via un mélangeur planétaire avec ou sans double enveloppe ; puis
- on a pulvérisé la solution colloïdale filmogène pendant 3 minutes, sur ce pré-mélange d'excipients en mouvement ; puis
- on a mesuré l'humidité résiduelle des particules ainsi revêtues, qui était de l'ordre de 7 % ; puis
- on a séché pendant 5 heures en étuve, à une température de 50° C, les particules du pré-mélange revêtues de cette solution colloïdale, suite à la pulvérisation ; puis
- on a mesuré l'humidité résiduelle des particules revêtues et séchées, qui était de l'ordre de 2,6 % ; puis
- on a procédé à la dispersion par mélangeage du principe actif, en lubrifiant l'ensemble.

On notera que l'absence de granulation, qui caractérise la pulvérisation mise en oeuvre pour l'obtention de la composition 3, se traduit par une densité apparente de cette composition 3 avant compression qui est inchangée suite à la pulvérisation et au séchage.

On notera également que l'on pourrait utiliser pratiquement le même protocole, pour l'obtention d'une composition dans laquelle la pulvérisation de la solution colloïdale serait directement effectuée sur les particules solides incorporant le principe actif en plus des excipients.

Après compression, on a obtenu des comprimés 3 selon l'invention à délitement rapide en bouche, qui contiennent chacun 1 mg de Rispéridone à titre de principe actif.

Chaque comprimé 3 présente une masse finale de 100 mg, une dureté moyenne de 21 Newtons, une épaisseur de 2,2 mm et une friabilité de l'ordre de 0,45 %.

### b) Essais in vivo d'hydratation de ces comprimés 3 :

On a procédé à des essais d'hydratation sur six individus humains. On a déterminé la vitesse d'hydratation de ces comprimés 3 selon l'invention, définie ici comme étant la durée nécessaire pour obtenir la désintégration ou délitement en bouche de ces comprimés par l'action de la salive, en réalisant une moyenne sur les six individus. On a ainsi obtenu pour ces comprimés 3 une vitesse d'hydratation de 25 secondes.

### 3) Comprimés 6 selon l'invention :

### a) Préparation des comprimés 6 :

On a préparé des compositions pharmaceutiques 6 pulvérulentes selon l'invention d'une manière analogue à celle décrite au § 2) ci-dessus. Chaque composition 6 présente la formulation suivante à l'état sec (en fractions massiques dans le tableau 7 ci-après) :

**Tableau 7 :**

| COMPOSITION | 6 |
|---|---|
| Pré-mélange de particules d'excipients revêtu par pulvérisation : | |
| Erythritol (diluant) | 59,87 % |
| Arôme fraise | 1,5 % |
| Aspartame | 1 % |
| « PRUV » (lubrifiant anti-collant pour la mise en oeuvre) | 2 % |
| cellulose microcristalline « AVICEL Ph 200 » (agent de cohésion) | 20 % |
| Film d'une microémulsion aqueuse selon l'invention après séchage | |
| à base d'eau et du système auto-émulsionnable suivant : | |
| « PEG 1500 » (composé hydrophile filmogène) | 1 % |
| « GELUCIRE 50/13 » (composé amphiphile humectant) | 5,5 % |
| Stéarate de magnésium (composé amphiphile lubrifiant) | 2 % |
| Lauryl sulfate de sodium (composé amphiphile lubrifiant) | 1 % |
| Lopéramide (principe actif) | 6,13 % |

On notera que l'absence de granulation, qui caractérise la pulvérisation mise en oeuvre pour l'obtention de la composition 6, se traduit par une densité apparente de cette composition 6 avant compression qui est inchangée suite à la pulvérisation et au séchage.

Après compression, on a obtenu des comprimés 6 selon l'invention à délitement rapide en bouche, qui contiennent chacun 2 mg de Lopéramide à titre de principe actif.

Chaque comprimé 6 présente une masse finale de 166 mg, une dureté moyenne de 22 Newtons, une épaisseur de 2,08 mm et une friabilité de l'ordre de 0,75 %.

### b) Essais in vivo d'hydratation de ces comprimés 6 :

On a procédé à des essais d'hydratation sur six individus humains, en procédant comme décrit au § 2) ci-dessus. On a ainsi obtenu pour ces comprimés 6 une vitesse d'hydratation de 19 secondes.

### 4) Comprimés 10 selon l'invention :

### a) Préparation des comprimés 10 :

On a préparé des compositions pharmaceutiques 10 pulvérulentes selon l'invention d'une manière analogue à celle décrite au § 2) ci-dessus. Chaque composition 10 présente la formulation suivante à l'état sec (en fractions massiques dans le tableau 11 ci-après) :

**Tableau 11 :**

| COMPOSITION | 10 |
|---|---|
| Pré-mélange de particules d'excipients revêtu par pulvérisation : | |
| Mannitol (diluant) | 32,77 % |
| Sorbitol (diluant) | 15 % |
| Arôme menthe | 1 % |
| Aspartame | 2 % |
| cellulose microcristalline « AVICEL Ph 200 » (agent de cohésion) | 10 % |
| Film d'une microémulsion aqueuse selon l'invention après séchage | |
| à base d'eau et du système auto-émulsionnable suivant : | |
| « PEG 1500 » (composé hydrophile filmogène) | 3 % |
| « Poloxamer 188 » (composé amphiphile humectant) | 0,6 % |
| « Stéarate de magnésium » (composé amphiphile lubrifiant) | 2 % |
| « GELUCIRE 44/14 » (composé amphiphile humectant) | 0,3 % |
| Ibuprofène (principe actif) | 33,33 % |

On notera que l'absence de granulation, qui caractérise cette pulvérisation, se traduit par une densité apparente de cette composition 10 avant compression qui est inchangée suite à la pulvérisation et au séchage.

Après compression, on a obtenu des comprimés 10 selon l'invention à délitement rapide en bouche, qui contiennent chacun 200 mg d'Ibuprofène à titre de principe actif. Chaque comprimé 10 présente une masse finale de 600 mg, une dureté moyenne de 21 Newtons, une épaisseur de 2,07 mm et une friabilité de l'ordre de 0,7 %.

### b) Essais in vivo d'hydratation de ces comprimés 10 :

On a procédé à des essais d'hydratation sur six individus humains, en procédant comme décrit au § 2) ci-dessus. On a ainsi obtenu pour ces comprimés 10 une vitesse d'hydratation de 28 secondes.

## Revendications

1. Composition pharmaceutique pulvérulente utilisée pour constituer après compression une forme galénique à délitement rapide en bouche, ladite composition comprenant des particules solides à base d'un mélange d'excipients qui comprend au moins un diluant choisi parmi une dextrine, une maltodextrine, le lactose, le saccharose, le tréhalose, le mannitol, le sorbitol, l'érythritol, le maltitol, le lactitol et le fructose, lesdites particules étant revêtues d'un film à base d'une solution colloïdale aqueuse qui est prévu pour pré-hydrater ladite composition, de telle sorte que lesdites particules ainsi revêtues soient libres les unes par rapport aux autres dans ladite composition, ladite solution colloïdale comprenant un système auto-émulsionnable comprenant en combinaison au moins un composé filmogène hydrophile, au moins un composé amphiphile lubrifiant, et au moins un composé amphiphile humectant, dans laquelle :
- ledit au moins un composé filmogène hydrophile est un carbohydrate choisi dans le groupe constitué par les maltodextrines, les dextrines, le sorbitol, le mannitol et le xylitol ou un polyéthylène glycol de masse moléculaire moyenne en poids Mw allant de 1000 g/mol à 6000 g/mol,
- ledit au moins un composé amphiphile lubrifiant est choisi dans le groupe constitué par le lauryl éther sulfate de sodium, le lauryl sulfate de sodium, et le stéarate de magnésium, et
- ledit au moins un composé amphiphile humectant est choisi parmi un lauroyl macrogolglycéride, tel qu'un lauroyl macrogol-32 glycéride (Gelucire^{®}44/14), et un stéaroyl macrogolglycéride, tel qu'un stéaroyl macrogol-32 glycéride (Gelucire^{®}50/13),
et dans laquelle le rapport massique solution colloïdale/particules destinées à recevoir ladite solution colloïdale est inférieur ou égal à 10%.

2. Composition pharmaceutique selon la revendication 1, **caractérisée en ce que** la fraction massique de ladite solution colloïdale à l'état sec dans ladite composition appartient à un domaine allant de 0,01 % à 10 %.

3. Composition pharmaceutique selon la revendication 2, **caractérisée en ce que** la fraction massique de ladite solution colloïdale à l'état sec dans ladite composition appartient à un domaine allant de 0,1 % à 5 %.

4. Composition pharmaceutique selon la revendication 3, **caractérisée en ce que** la fraction massique de ladite solution colloïdale à l'état sec dans ladite composition appartient à un domaine allant de 0,5 % à 3 %.

5. Composition pharmaceutique selon une des revendications précédentes, **caractérisée en ce que** ledit ou chaque diluant est soluble dans l'eau, le(s)dit(s) diluant(s) étant présent(s) dans ladite composition selon une fraction massique totale allant de 30 % à 90 %.

6. Composition pharmaceutique selon la revendication 5, **caractérisée en ce que** ledit ou l'un au moins desdits diluants est un diluant soluble dans l'eau qui est choisi dans le groupe constitué par les dextrines et les maltodextrines.

7. Composition pharmaceutique selon la revendication 5, **caractérisée en ce que** ledit ou l'un au moins desdits diluants comprend un dérivé de sucres choisi dans le groupe constitué par le lactose, le saccharose, le tréhalose, le mannitol, le sorbitol, l'érythritol, le maltitol, le lactitol et le fructose.

8. Composition pharmaceutique selon l'une quelconque des revendications précédentes, **caractérisée en ce que** ledit mélange d'excipients comprend en outre au moins un agent de cohésion, tel que la cellulose microcristalline, les carboxyméthylcelluloses sodiques, les carboxyméthylcelluloses de calcium, les croscarmelloses, une dextrine, le lactose, le(s)dit(s) agent(s) de cohésion étant présent(s) dans ladite composition selon une fraction massique allant de 0,1 % à 40%.

9. Composition pharmaceutique selon l'une quelconque des revendications précédentes, **caractérisée en ce qu'**elle comprend au moins un principe actif à l'état dispersé dans ledit mélange d'excipients.

10. Forme galénique à délitement rapide en bouche et comprenant un comprimé non enrobé, **caractérisée en ce que** ledit comprimé est constitué d'une composition pharmaceutique selon une des revendications 1 à 9.

11. Procédé de fabrication d'une composition pharmaceutique selon l'une quelconque des revendications 1 à 9, **caractérisé en ce qu'**il comprend les étapes suivantes :
a) on prépare une solution colloïdale aqueuse de type huile dans eau ou eau dans huile comprenant, d'une part, une phase aqueuse et, d'autre part, un système auto-émulsionnable tel que défini dans la revendication 1,
b) on pulvérise la solution colloïdale obtenue en a) :
(i) sur des particules solides constituées d'un principe actif dispersé dans un mélange d'excipients comprenant au moins un diluant choisi parmi une dextrine, une maltodextrine, le lactose, le saccharose, le tréhalose, le mannitol, le sorbitol, l'érythritol, le maltitol, le lactitol et le fructose, ou
(ii) sur des particules solides seulement constituées dudit mélange d'excipients comprenant au moins un diluant choisi parmi une dextrine, une maltodextrine, le lactose, le saccharose, le tréhalose, le mannitol, le sorbitol, l'érythritol, le maltitol, le lactitol et le fructose,
de telle manière que le rapport massique (solution colloïdale pulvérisable/ particules destinées à recevoir ladite solution colloïdale) soit inférieur ou égal à 10%;
c) on sèche la solution colloïdale pulvérisée en b) pour l'obtention :
- dans le cas (i), de ladite composition pharmaceutique constituée desdites particules solides comprenant ledit principe actif qui sont revêtues d'un film ; ou
- dans le cas (ii), d'une composition intermédiaire constituée desdites particules solides d'excipients qui sont revêtues dudit film, puis
d) optionnellement, dans ce cas (ii), on disperse ledit principe actif dans ladite composition intermédiaire obtenue en c) pour l'obtention de ladite composition pharmaceutique.

12. Procédé selon la revendication 11, **caractérisé en ce que** ladite solution colloïdale préparée à l'étape a) comprend :
- une fraction massique dudit système auto-émulsionnable inférieure ou égale à 10 %, et
- une fraction massique d'eau égale ou supérieure à 90 %.

13. Procédé selon une des revendications 11 ou 12, **caractérisé en ce que** l'étape b) est mise en oeuvre à une température allant de 40° C à 70° C.

14. Procédé selon une des revendications 11 à 13, **caractérisé en ce que** le rapport de la densité apparente de la composition obtenue à l'étape c) ou d) sur la densité apparente des particules solides avant pulvérisation est compris entre 1 et 1,2.

## Patentansprüche

1. Eine pulverförmige Arzneizusammensetzung, die verwendet wird, um nach Verdichtung eine im Mund rasch zerfallende Dosierungsform zu bilden, wobei die Zusammensetzung Feststoffteilchen auf der Basis einer Trägerstoffmischung umfasst, die mindestens ein Verdünnungsmittel, ausgewählt aus einem Dextrin, einem Maltodextrin, Laktose, Saccharose, Trehalose, Mannitol, Sorbitol, Erythritol, Maltitol, Lactitol und Fructose, umfasst, wobei die Teilchen mit einem Film auf der Basis einer wässrigen kolloidalen Lösung überzogen sind, der zum Vorhydrieren der Zusammensetzung bestimmt ist, sodass die so überzogenen Teilchen in der Zusammensetzung in Bezug aufeinander ungebunden sind, wobei die kolloidale Lösung ein selbstemulgierendes System umfasst, das in Kombination mindestens eine hydrophile filmbildende Verbindung, mindestens eine schmierfähige amphiphile Verbindung und mindestens eine feuchtigkeitsspendende amphiphile Verbindung umfasst, wobei:
- die mindestens eine hydrophile filmbildende Verbindung ein Kohlenhydrat, ausgewählt aus der Gruppe, bestehend aus Maltodextrinen, Dextrinen, Sorbitol, Mannitol und Xylitol, oder ein Polyethylenglykol mit einem gewichtsmittleren Molekulargewicht Mw von 1000 g/mol bis 6000 g/mol ist,
- die mindestens eine schmierfähige amphiphile Verbindung ausgewählt ist aus der Gruppe, bestehend aus Natriumlaurylethersulfat, Natriumlaurylsulfat und Magnesiumstearat, und
- wobei die mindestens eine feuchtigkeitsspendende amphiphile Verbindung ausgewählt ist aus einem Lauroylmakroglycerid wie etwa einem Lauroylmakrogol-32-Glycerid (Gelucire^{®}44/14) und einem Stearoylmakroglycerid wie etwa einem Stearoylmakrogol-32-Glycerid (Gelucire^{®}50/13),
und wobei das Massenverhältnis kolloidale Lösung/Teilchen, die zum Aufnehmen der kolloidalen Lösung bestimmt sind, kleiner als oder gleich 10 % ist.

2. Arzneizusammensetzung gemäß Anspruch 1, **dadurch gekennzeichnet, dass** der Massenanteil der kolloidalen Lösung im trockenen Zustand in der Zusammensetzung in einem Bereich von 0,01 % bis 10 % liegt.

3. Arzneizusammensetzung gemäß Anspruch 2, **dadurch gekennzeichnet, dass** der Massenanteil der kolloidalen Lösung im trockenen Zustand in der Zusammensetzung in einem Bereich von 0,1 % bis 5 % liegt.

4. Arzneizusammensetzung gemäß Anspruch 3, **dadurch gekennzeichnet, dass** der Massenanteil der kolloidalen Lösung im trockenen Zustand in der Zusammensetzung in einem Bereich von 0,5 % bis 3 % liegt.

5. Arzneizusammensetzung gemäß einem der vorhergehenden Ansprüche, **dadurch gekennzeichnet, dass** das oder jedes Verdünnungsmittel in Wasser löslich ist, wobei das/die Verdünnungsmittel in der Zusammensetzung gemäß einem Gesamtmassenanteil von 30 % bis 90 % vorhanden ist/sind.

6. Arzneizusammensetzung gemäß Anspruch 5, **dadurch gekennzeichnet, dass** das Verdünnungsmittel oder mindestens eines der Verdünnungsmittel ein in Wasser lösliches Verdünnungsmittel ist, das aus der Gruppe, bestehend aus Dextrinen und Maltodextrinen, ausgewählt ist.

7. Arzneizusammensetzung gemäß Anspruch 5, **dadurch gekennzeichnet, dass** das Verdünnungsmittel oder mindestens eines der Verdünnungsmittel ein Derivat von Zuckern, ausgewählt aus der Gruppe, bestehend aus Lactose, Saccharose, Trehalose, Mannitol, Sorbitol, Erythritol, Maltitol, Lactitol und Fructose, umfasst.

8. Arzneizusammensetzung gemäß einem der vorhergehenden Ansprüche, **dadurch gekennzeichnet, dass** die Trägerstoffmischung ferner mindestens ein Bindemittel wie etwa mikrokristalline Cellulose, Natrium-Carboxymethylcellulosen, Calcium-Carboxymethylcellulosen, Croscarmellosen, ein Dextrin, Lactose umfasst, wobei das/die Bindemittel in der Zusammensetzung mit einem Massenanteil von 0,1 % bis 40 % vorhanden ist/sind.

9. Arzneizusammensetzung gemäß einem der vorhergehenden Ansprüche, **dadurch gekennzeichnet, dass** sie mindestens einen aktiven Bestandteil im in der Trägerstoffmischung dispergierten Zustand umfasst.

10. Eine Dosierungsform, die im Mund rasch zerfällt und die eine nicht umhüllte Tablette umfasst, **dadurch gekennzeichnet, dass** die Tablette aus einer Arzneizusammensetzung gemäß einem der Ansprüche 1 bis 9 besteht.

11. Ein Verfahren zur Herstellung einer Arzneizusammensetzung gemäß einem der Ansprüche 1 bis 9, **dadurch gekennzeichnet, dass** es die folgenden Schritte umfasst:
a) Zubereiten einer wässrigen kolloidalen Lösung des Typs Öl in Wasser oder Wasser in Öl, die einerseits eine wässrige Phase und andererseits ein selbstemulgierendes System, wie in Anspruch 1 definiert, umfasst,
b) Sprühen der in a) erhaltenen kolloidalen Lösung:
(i) auf Feststoffteilchen, die aus einem aktiven Bestandteil bestehen, der in einer Trägerstoffmischung dispergiert ist, umfassend mindestens ein Verdünnungsmittel, ausgewählt aus einem Dextrin, einem Maltodextrin, Lactose, Saccharose, Trehalose, Mannitol, Sorbitol, Erythritol, Maltitol, Lactitol und Fructose, oder
(ii) auf Feststoffteilchen, die nur aus der Trägerstoffmischung bestehen, umfassend mindestens ein Verdünnungsmittel, ausgewählt aus einem Dextrin, einem Maltodextrin, Lactose, Saccharose, Trehalose, Mannitol, Sorbitol, Erythritol, Maltitol, Lactitol und Fructose,
sodass das Massenverhältnis (sprühbare kolloidale Lösung/Teilchen, die zum Aufnehmen der kolloidalen Lösung bestimmt sind) kleiner als oder gleich 10 % ist;
c) Trocknen der in b) gesprühten kolloidalen Lösung zum Erhalten von:
- im Fall (i) der Arzneizusammensetzung, die aus den aktiven Bestandteil umfassenden Feststoffteilchen besteht, die mit einem Film überzogen sind; oder
- im Fall (ii) einer Zwischenzusammensetzung, die aus den Trägerstoff-Feststoffteilchen, die mit dem Film überzogen sind, besteht, dann
d) wahlweise, im Fall (ii) Dispergieren des aktiven Bestandteils in der Zwischenzusammensetzung, die in c) erhalten wurde, zum Erhalten der Arzneizusammensetzung.

12. Verfahren gemäß Anspruch 11, **dadurch gekennzeichnet, dass** die in Schritt a) zubereitete kolloidale Lösung Folgendes umfasst:
- einen Massenanteil des selbstemulgierenden Systems, der kleiner als oder gleich 10 % ist, und
- einen Massenanteil von Wasser, der gleich oder größer als 90 % ist.

13. Verfahren gemäß einem der Ansprüche 11 oder 12, **dadurch gekennzeichnet, dass** Schritt b) bei einer Temperatur von 40 °C bis 70 °C ausgeführt wird.

14. Verfahren gemäß einem der Ansprüche 11 bis 13, **dadurch gekennzeichnet, dass** das Verhältnis der scheinbaren Dichte der in Schritt c) oder d) erhaltenen Zusammensetzung zu der scheinbaren Dichte der Feststoffteilchen vor dem Besprühen zwischen 1 und 1,2 liegt.

## Claims

1. A powdery pharmaceutical composition used to constitute, after compression, a dosage form which breaks down quickly in the mouth, said composition comprising solid particles based on a mixture of excipients which comprises at least one diluent chosen from a dextrin, a maltodextrin, lactose, saccharose, trehalose, mannitol, sorbitol, erythritol, maltitol, lactitol and fructose, said particles being coated with a film based on an aqueous colloidal solution provided to prehydrate said composition, such that said particles thus coated are free from one another in said composition, said colloidal solution comprising a self-emulsifiable system comprising in combination at least one hydrophilic film-forming compound, at least one lubricating amphiphilic compound, and at least one moistening amphiphilic compound, wherein:
- said at least one hydrophilic film-forming compound is a carbohydrate chosen from the group constituted by maltodextrins, dextrins, sorbitol, mannitol and xylitol or a polyethylene glycol with a weight average molecular mass Mw ranging from 1000 g/mol to 6000 g/mol,
- said at least one lubricating amphiphilic compound is chosen from the group constituted by sodium lauryl ether sulfate, sodium lauryl sulfate, and magnesium stearate, and
- said at least one moistening amphiphilic compound is chosen from a lauroyl macrogolglyceride, such as a lauroyl macrogol-32 glyceride (Gelucire^{®}44/14), and a stearoyl macrogolglyceride, such as a stearoyl macrogol-32 glyceride (Gelucire^{®}50/13)
and wherein the mass ratio of colloidal solution/particles intended to receive said colloidal solution is less than or equal to 10%.

2. The pharmaceutical composition according to Claim 1, **characterised in that** the mass fraction of said colloidal solution in dry state in said composition is in a range from 0.01% to 10%.

3. The pharmaceutical composition according to Claim 2, **characterised in that** the mass fraction of said colloidal solution in dry state in said composition is in a range from 0.1 % to 5%.

4. The pharmaceutical composition according to Claim 3, **characterised in that** the mass fraction of said colloidal solution in dry state in said composition is in a range from 0.5% to 3%.

5. The pharmaceutical composition according to one of the preceding claims, **characterised in that** said or each diluent is water-soluble, said diluent(s) being present in said composition in a total mass fraction ranging from 30% to 90%.

6. The pharmaceutical composition according to Claim 5, **characterised in that** said diluent or at least one of said diluents is a water-soluble diluent which is chosen from the group constituted by dextrins and maltodextrins.

7. The pharmaceutical composition according to Claim 5, **characterised in that** said diluent or at least one of said diluents comprises a sugar derivative chosen from the group constituted by lactose, saccharose, trehalose, mannitol, sorbitol, erythritol, maltitol, lactitol and fructose.

8. The pharmaceutical composition according to any one of the preceding claims, **characterised in that** said mixture of excipients further comprises at least one cohesion agent, such as microcrystalline cellulose, sodium carboxymethylcelluloses, calcium carboxymethylcelluloses, croscarmelloses, a dextrin, lactose, said cohesion agent(s) being present in said composition in a mass fraction ranging from 0.1 % to 40%.

9. The pharmaceutical composition according to any one of the preceding claims, **characterised in that** it comprises at least one active ingredient in a dispersed state in said mixture of excipients.

10. A dosage form which breaks down quickly in the mouth and which comprises a non-coated tablet, **characterised in that** said tablet is constituted by a pharmaceutical composition according to one of Claims 1 to 9.

11. A method of producing a pharmaceutical composition according to any one of Claims 1 to 9, **characterised in that** it comprises the following steps:
a) preparing an oil-in-water-type or water-in-oil-type aqueous colloidal solution comprising, on the one hand, an aqueous phase and, on the other hand, a self-emulsifiable system as defined in Claim 1,
b) spraying the colloidal solution obtained in a):
(i) onto solid particles constituted by an active ingredient dispersed in a mixture of excipients comprising at least one diluent selected from a dextrin, a maltodextrin, lactose, saccharose, trehalose, mannitol, sorbitol, erythritol, maltitol, lactitol and fructose, or
(ii) onto solid particles solely constituted by said mixture of excipients comprising at least one diluent chosen from a dextrin, a maltodextrin, lactose, saccharose, trehalose, mannitol, sorbitol, erythritol, maltitol, lactitol and fructose,
such that the mass ratio (sprayable colloidal solution/particles intended to receive said colloidal solution) is less than or equal to 10%;
c) drying the colloidal solution sprayed in b) to obtain:
- in case (i), said pharmaceutical composition constituted by said solid particles comprising said active ingredient which are coated with a film; or
- in case (ii), an intermediate composition constituted by said solid particles of excipients which are coated by said film, then
d) optionally, in this case (ii), dispersing said active ingredient in said intermediate composition obtained in c) to obtain said pharmaceutical composition.

12. The method according to Claim 11, **characterised in that** said colloidal solution prepared in step a) comprises:
- a mass fraction of said self-emulsifiable system of less than or equal to 10%, and
- a mass fraction of water equal to or greater than 90%.

13. The method according to one of Claims 11 or 12, **characterised in that** step b) is performed at a temperature ranging from 40°C to 70°C.

14. The method according to one of Claims 11 to 13, **characterised in that** the ratio of the apparent density of the composition obtained in step c) or d) to the apparent density of the solid particles before spraying is between 1 and 1.2.
